# EUROPEAN PATENT APPLICATION

(11) **EP 3 673 795 A1**
(43) Date of publication of application: **01.07.2020**
(21) Application number: 19220038.4
(22) Date of filing: 30.12.2019
(51) Int. Cl.: A61B 5/00, G01N 21/00

(54) **A DEVICE FOR PROTECTION OF A FIBRE-OPTIC SENSOR FOR MEDICAL APPLICATIONS**

(30) Priority: 28.12.2018 EP 18248263
(71) Applicant: SDS Optic Spolka Akcyjna, 20-708 Lublin (PL)
(72) Inventor: Staniszewska, Magdalena, 21-030 Motycz (PL); Staniszewski, Marcin, 21-030 Motycz (PL)
(74) Representative: Kondrat, Mariusz

(57) **Abstract**

The subject of the invention is a device protecting a fibre-optic sensor for medical applications, characterized in that the device is a capillary open on both sides (1) with an internal diameter between 1 µm and 3 mm and an external diameter between 2 µm and 5 mm.

## Description

The subject of the invention is a device protecting the fibre-optic sensor for medical applications.

The state of the art describes a biosensor-based toxin detection device for milk samples in which the surface of a Fabry-Perot interferometer, which is a capillary-flow glass microplate, has been coated with fragments of antibodies, the so-called Fab' (Chalyan T et al., Asymmetric Mach-Zehnder Interferometer Based Biosensors for Aflatoxin M1 Detection, Biosensors 2016, 6, 1; doi:10.3390/bios6010001).

The US 10/483,586 application (publication no. US20040186359 A) describes a diagnostic apparatus using detection by means of a coated optical fibre; which, however, requires implantation in the body.

The Clinical Chemistry publication of 1991, entitled Evaluation of the Fiber-Optic Antibody-Based Fluoroimmunosensor for DNA Adducts in Human Placenta Samples by T. Vo-Dinh, J. P. Alarie, R. W. Johnson, M. J. Sepaniak and R. M. Santella describes the measurement of DNA adducts by means of fluorescent signal detection by a single optical fibre with antibodies placed in a pocket at the end of the fibre. The device uses laser light to excite and detect the fluorescent signal. The measurement of adducts requires preparation of the sample by hydrolysis in order to achieve the required sensitivity.

Similarly, the apparatus and method of measurement presented in Biosensors and Bioelectronics 2007, Chemiluminescent optical fiber immunosensor for detection of autoantibodies to ovarian and breast cancer-associated antigens by Orly Salama, Sebastien Herrmann, Alina Tziknovsky b, Benjamin Piura, Michael Meirovich, Ilya Trakht, Brent Reed, Leslie I. Lobel, Robert S. Marks requires a marker, e.g. a chemiluminescent one and secondary antibodies for detection. The device is prepared to perform measurements in a solution.

The document Nature Biotechnology 2000, Antibody-based nanoprobe for measurement of a fluorescent analyte in a single cell by Tuan Vo-Dinh, Jean-Pierre Alarie, Brian M. Cullum, and Guy D. Griffin describes another apparatus using an optical fibre coated with antibodies, suitable for use in a single cell. In this device, however, measurement is also based on detection of the fluorescent signal and the device includes a single optical fibre connected to a fluorescence microscope.

The state of the art has already indicated some solutions enabling elimination of the use of markers for detection. The Scientific Reports publication of 2014, entitled Nanoscale Label-free Bioprobes to Detect Intracellular Proteins in Single Living Cells by Wooyoung Hong, Feng Liang, Diane Schaak, Marko Loncar and Qimin Quan describes an apparatus using an antibody-coated optical fibre that does not require fluorescent markers and is based on the surface plasmon resonance technique. The device has been adapted to perform measurements in a single cell and includes an optical fibre terminated with an antibody-coated gold nanotube. When the analyte is connected, the LSPR signal is shifted. Similarly, a Biosensors and Bioelectronics document of 2014, entitled An enhanced LSPR fiber-optic nanoprobe for ultrasensitive detection of protein biomarkers by Mollye Sanders, Yongbin Lin, Jianjun Wei, Taylor Bono, Robert G. Lindquist describes an analogous solution, also based on an optical fibre probe and the use of the LSPR signal from an antibody-coated gold nanodisc at the end of the probe. The device is adapted to determine the analyte in a solution. There are no solutions described to carry out measurements directly in the tissue.

Currently known solutions do not enable a simple, minimally invasive determination of analytes directly in tissues, including solid tissues, among others, a solid tumour, whether isolated or in situ directly in the patient, without having to use any markers and without prior tissue preparation. Current diagnostic solutions require sampling, fixation and/or processing and therefore do not reflect the actual condition of the tissue. In addition, devices using the flow of the tested substance through the device (as in the case of microplates) require significant amounts of the sample. The use of a microprobe used for in-situ measurements ensures safety in use and limits the amount of material required for the assay.

A device known from the description of the P.420189 application contains an optical sensor that uses selective interaction with the tested substance of the binding agents immobilised on the surface of the optical fibre which is the interferometer arm, where the optical fibre being the interferometer arm, containing the immobilized binding agent, is fixed inside a guide, preferably a metal one, enabling piercing of the tissue and direct measurement of the substance present in the tissue, so as to detect it and/or determine the concentration. Design of the device according to the invention , enables to insert a fibre-based sensor without interfering with the measurement and damaging the sensor itself, directly into the tissue. This device also enables in-situ measurement with an optical fibre-based probe in a safe and minimally invasive way for the patient. The guide also makes it possible to protect the patient from the remaining part of the fibre-optic sensor in the tissue, e.g. should the sensor accidentally crack or break. The optical fibre may be fixed in the guide and thus shielded in part or over its entire length to reduce the effect of any shocks on the measurement result.

The purpose of the invention was to provide a device for protection of a fibre-optic sensor for medical applications.

The essence of the invention is a device protecting a fibre-optic medical sensor, characterized in that the device is a capillary open on both sides with an internal diameter between 1 µm and 3 mm and an external diameter between 2 µm and 5 mm.

The capillary is preferably made of a material selected from a group including metal, glass or plastic.

Preferably, when the sensor is mounted in the capillary, the front of the sensor is extended beyond the capillary to a distance of 0 to 5 mm.

Preferably the face of the sensor is flush with the face of the capillary.

Preferably, the front of the sensor is retracted inwards in the capillary from 0 to 30 mm, measuring from the front of the capillary.

The invention provides the following advantages:
- protection of the fibre-optic sensor from mechanical damage when placing the fibre-optic sensor in the patient's body;
- mechanical protection of the sensor during transport against breaking and other mechanical damage such as abrasion;
- mechanical protection of the sensor during its use in measuring the specific substance against breaking and other mechanical damage, such as abrasion;
- use of the capillary effect for the seepage of the measured substances into the sensor face.

The invention is shown in figures, where fig. 1 shows the top view of the capillary; fig. 2 shows the bottom view of the capillary; fig. 3 is a schematic presentation of the apparatus according to the invention with the fibre-optic sensor installed in it, in the option where the face of the sensor protrudes outside of the capillary; fig. 4 is a schematic presentation of the apparatus according to the invention with the fibre-optic sensor installed in the option where the face of the sensor is flush with the face of the capillary; fig. 5 is a schematic presentation of the apparatus according to the invention with the fibre-optic sensor installed in it in the option where the front of the sensor is inside the capillary and is retracted from the front of the capillary; fig. 6 is a schematic presentation of the apparatus according to the invention with the fibre-optic sensor installed in it, in the option where the internal wall of the sensor is located from 0 µm to 1.499 mm from the internal wall of the capillary.

The invention is presented in embodiments.

### Embodiment 1

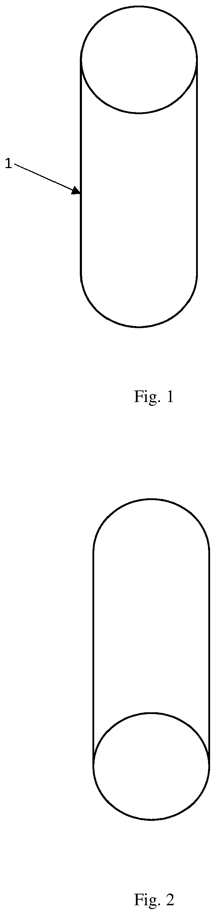

The protective device for the fibre-optic sensor for medical applications according to the invention is shown in fig. 1-6, where 1 is the capillary; 2 is the front of the capillary; 3 is the fibre-optic sensor; 4 is the front of the fibre-optic sensor; 5 is the wall of the capillary and "a" is the distance between the optical fibre and the wall of the capillary.

The device according to the invention is characterised by the fact that it is a double-sided open capillary 1 with an inner diameter between 1 µm and 3 mm and an outer diameter between 2 µm and 5 mm.

Capillary 1 can be made of a material chosen from a group including metal, glass or plastic.

In this embodiment, the capillary indicated in fig. 1-2 was made by a device such as the Microelectrode Puller from World Precision Instruments. The fabrication of the capillary uses processes such as drawing, heating, cooling.

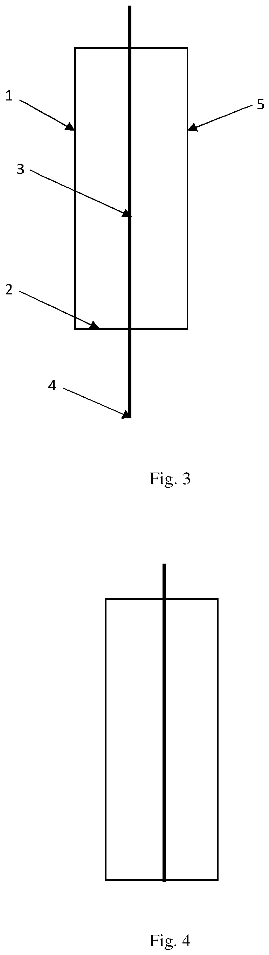

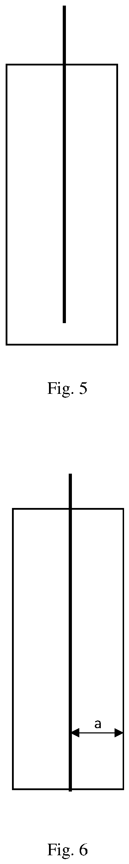

The solution according to the invention provides the following options of placing the fibre-optic sensor 3 in capillary 1:
a) option 1 (fig. 3), where the front 4 of the fibre-optic sensor 3 is extended beyond capillary 1 to a distance of 0 mm to 5 mm from the front of capillary 2;
b) option 2 (fig. 4), where the front 4 of the fibre-optic sensor 3 is flush with the front of capillary 2;
c) option 3 (fig. 5), where the front 4 of the fibre-optic sensor 3 is located inside the capillary and is retracted between 0 mm and 30 mm from the front of the capillary.

Whereas in any of the options described above the other end of the sensor protrudes freely from the other end of the capillary.

Whereas the optical fibre with the sensor (i.e. the fibre-optic sensor 3) is placed in capillary 1 in such a way that the outer wall of the sensor is placed at a distance "a" between 0 µm and 1.499 mm from the inner wall 5 of the capillary (fig. 6).

Where the fibre-optic sensor may be any fibre-optic sensor.

## Claims

1. A device protecting a fibre-optic sensor for medical applications, **characterized in that** the device is a capillary open on both sides (1) with an internal diameter between 1 µm and 3 mm and an external diameter between 2 µm and 5 mm.

2. The device according to claim 1 **characterized in that** the capillary (1) is made of a material selected from a group including metal, glass or plastic.

3. The device according to claim 1 or 2, **characterized in that** when the sensor (3) is mounted in the capillary (1), the front of the sensor (4) is extended beyond the capillary at a distance of 0 to 5 mm.

4. The device according to claim 3, **characterized in that** the front of the fibre-optic sensor (4) is flush with the front of capillary (2).

5. The device according to claim 1 or 2 **characterized in that** the front of the sensor 4 is retracted inwards in the capillary 1 from 0 to 30 mm, measuring from the front of the capillary 2.
